(19) Europäisches Patentamt
European Patent Office
Office européen des brevets

(11) Publication number: **0 186 967 A1**

(12) # EUROPEAN PATENT APPLICATION

(21) Application number: **85308565.2**

(22) Date of filing: **26.11.85**

(51) Int. Cl.⁴: **C 07 D 233/16**, C 07 C 103/44, D 06 M 13/46, C 11 D 1/62

(30) Priority: **30.11.84 GB 8430301**

(43) Date of publication of application: **09.07.86 Bulletin 86/28**

(84) Designated Contracting States: **BE CH DE FR GB IT LI NL SE**

(71) Applicant: **THOMAS SWAN AND CO., LTD., Consett, Co. Durham DH8 7ND (GB)**

(72) Inventor: **Richardson, Frank Barnett, Derwent Hill Cottage East Law, Ebchester Consett, Co. Durham (GB)**

(74) Representative: **Overbury, Richard Douglas et al, HASELTINE LAKE & CO Hazlitt House 28 Southampton Buildings Chancery Lane, London WC2A 1AT (GB)**

(54) **Improvements in or relating to compositions of matter suitable for use as fabric softeners.**

(57) Aminopropyl ethylene diamine is reacted with a fatty acid or amide-forming derivative thereof to form a fatty diamide having a secondary amine group. The secondary amine group is then converted into a tertiary amine group by imidazoline formation or by alkoxylation and the tertiary amine group is quaternised. The resultant product includes an aminopropyl group which imparts improved fabric softening properties.

EP 0 186 967 A1

# Improvements in or relating to compositions of matter suitable for use as fabric softeners

This invention relates to compositions of matter suitable for use as fabric softeners.

It has long been known that certain cationic amine functional compounds confer what is commonly referred to as "softness" to various fabrics. In addition, to providing softness, the treatment of the fabrics with these cationic chemicals also confers anti-static properties to the fibres whether they are synthetic or natural fibres. The types of cationic chemicals which have been proposed for this purpose are exemplified below:-

Type 1: Alkyl dimethyl quaternary ammonium compounds

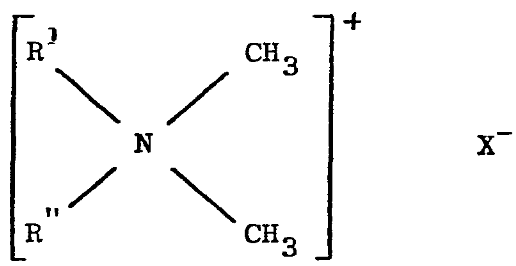

where R' and R" are fatty radicals having from 12 to 18 carbon atoms and where $X^-$ is halide, preferably chloride, or metho sulphate anion.

Type 2: Quaternised amido imidazolines

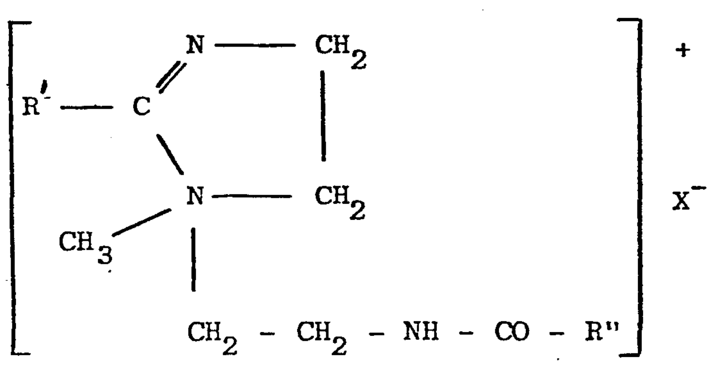

where R' and R" and $X^-$ have the meanings given above.

<u>Type 3: Quaternised alkoxylated fatty amides</u>

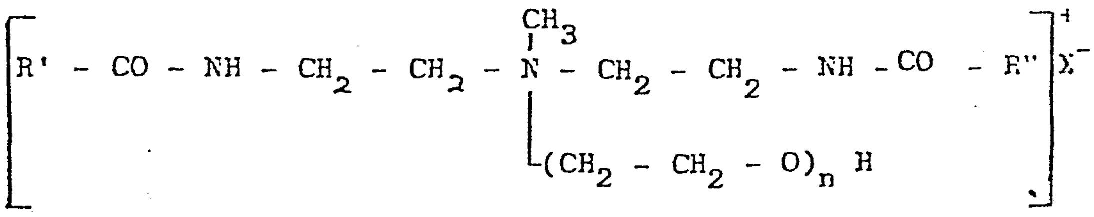

where R', R" and X have the meanings given above.

Most cationic compounds similar to those described above will confer some degree of softness onto a fabric. However, they can and do differ in other properties e.g. ease of application, cost, re-wetting properties, dispersibility. properties, softening power and anti-static properties.

In general most cationic softeners are based on either a di-hydrogenated tallow amine or an amido imidazoline based on diethylene triamine , both quaternised with either methyl chloride or dimethyl sulphate .. Previous work has already shown that an alkyl chain length of $C_{16}$ to $C_{18}$ is preferred, that straight chains are better than branched chains, and that saturated alkyl radicals are preferred to unsaturated radicals. However, none of the hitherto described fabric softeners is entirely satisfactory.

It is an object of the present invention to provide novel compositions of matter which have improved properties as fabric softeners.

According to one aspect of the present invention there is provided a composition of matter having the general formula

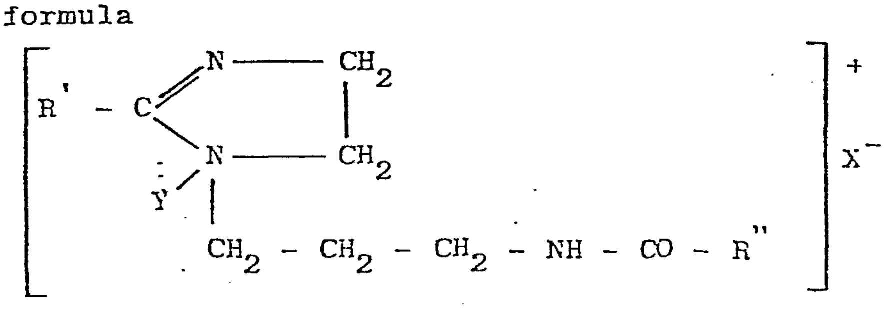

wherein R' and R" are fatty radicals having from 12 to 18 carbon atoms, Y is alkyl or aryl, and $X^-$ is a halide or a metho sulphate ion.

According to another aspect of the present invention there is provided a composition of matter having the general formula

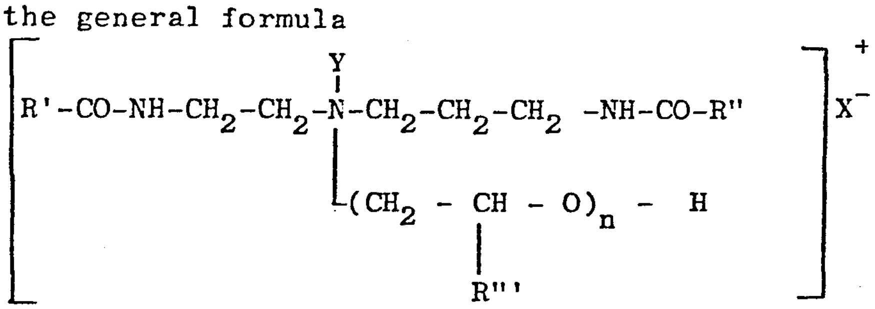

where R', R", $X^-$ and Y have the meanings given above, R"' is a hydrogen atom or a methyl group, and n is from 1 to 10.

According to a further aspect of the present invention there is provided a composition of matter which is the product obtained by reacting together aminopropyl ethylene diamine with a fatty acid or amide-forming derivative thereof to form a fatty diamide having a secondary amine group, converting the secondary amine group into a tertiary amine group by imidazoline formation or by alkoxylation, and quaternising the tertiary amine group.

Although the compositions of the present invention have improved softening power, they generally also have other advantages such as easier handling, better dispersing properties, better re-wettability properties and the ability to produce higher active formulations without associated viscosity problems. In addition, these improved properties can be achieved at a cost which is no greater than that of conventional fabric softeners.

The compositions of the present invention are either quaternary ammonium derivatives of fatty amino propyl imidazolines or quaternary ammonium derivatives of

alkoxylated amino propyl amino ethyl fatty amides.

Specific examples of the novel compositions of matter of the present invention are as follows:-

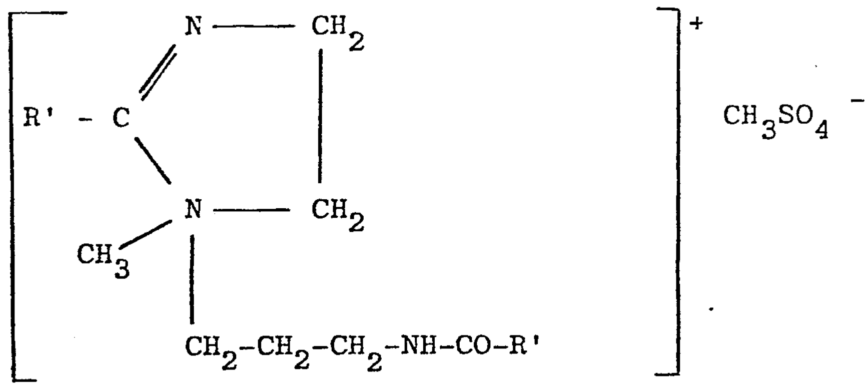

where R' is an oleic or stearyl radical.

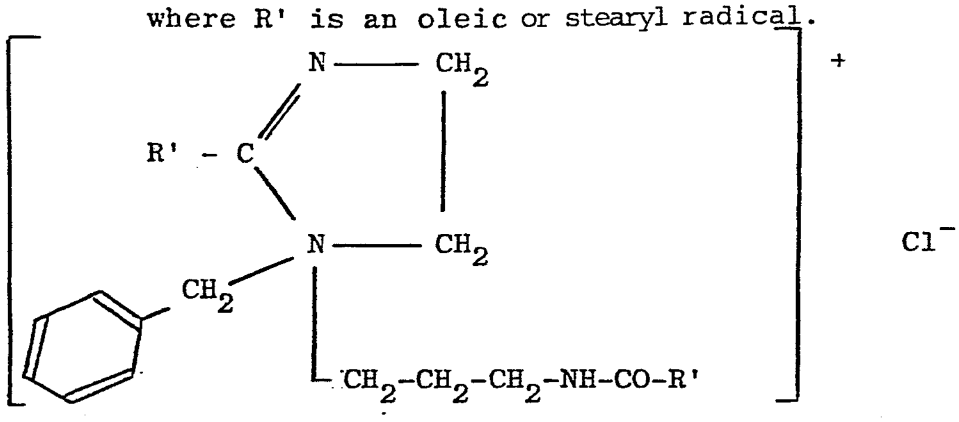

where R' is an oleic radical.

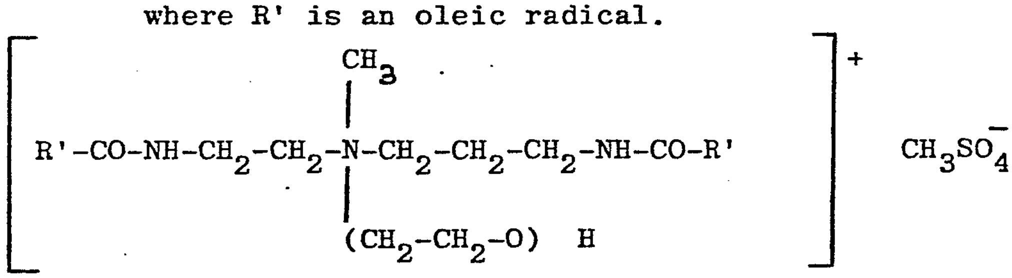

where R' is an oleic or stearyl radical.

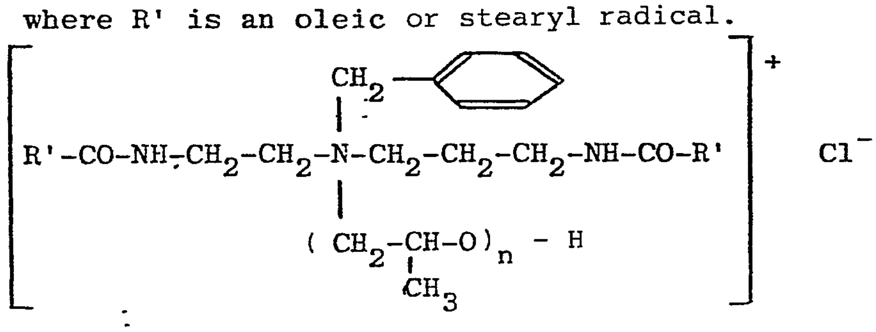

where R' is an oleic or stearyl radical.

The presence of the amino propyl groups in the compositions of the present invention confers very desirable properties to the composition insofar as their utility as fabric softeners is concerned. Generally, these novel compounds of the invention show the following advantages when compared with conventional fabric softening compounds:-

(a) Improved softness
(b) Lower set point
(c) Ability to produce dispersions in water at higher concentrations
(e) Improved re-wetting properties
(f) Improved cold water dispersibility

In addition, these novel compounds have acceptable cost and after-yellowing and anti-static characteristics.

Those compositions of the present invention which are quaternary ammonium derivatives of fatty acid amido propyl imidazolines may be prepared by reacting approximately one mole of amino propyl ethylene diamine with approximately two moles of fatty acid (or amide-forming derivative thereof such as the glyceride or other ester or the acid chloride) to produce a fatty diamide with the following structure.

$$R'-CO-NH-CH_2-CH_2-NH-CH_2-CH_2-CH_2-NH-CO-R''$$

where R' and R" are saturated or unsaturated fatty acid radicals of the same or different compositions and having from 12 to 18 carbon atoms.

This diamide is then further reacted to produce imidazoline formation with the liberation of water . The resultant imidazoline has the following structure:-

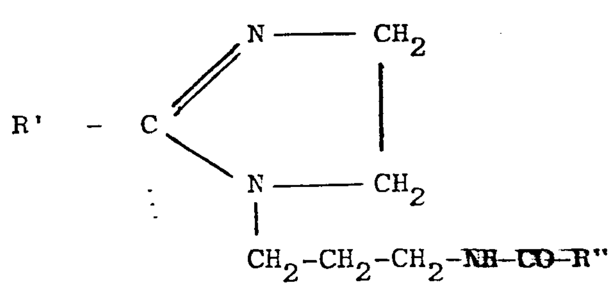

This amido propyl imidazoline is then quaternised by reaction with an alkylating agent of the type YX where Y is an alkyl or aryl group and X is a halide (preferably chloride) or metho sulphate group to form the desired composition of the structure:

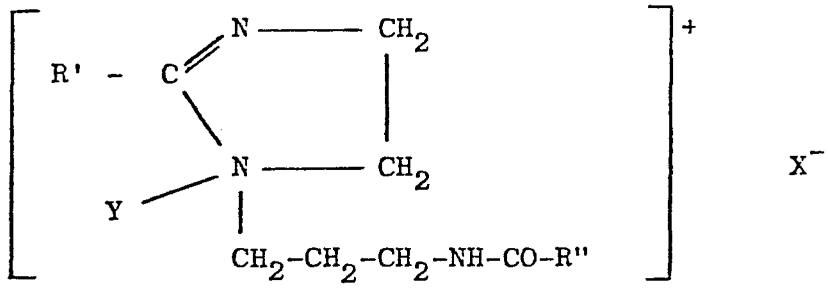

Those compositions of the present invention which are quaternary ammonium derivatives of alkoxylated amino propyl amino ethyl fatty amides may be prepared by reacting approximately one mole of amino propyl ethylene diamine with approximately two moles of fatty acid (or amide-forming derivative thereof) under conditions such as to prevent or reduce cyclisation to the imidazoline. The resultant diamide has the following structure :

$$R'-CO-NH-CH_2-CH_2-NH-CH_2-CH_2-CH_2-NH-CO-R''$$

where R' and R" have the meanings given above.

The next step is to further react this diamide with either ethylene oxide or propylene oxide, reaction taking place at the secondary amine group. The resultant alkoxylated fatty diamide has the following structure.

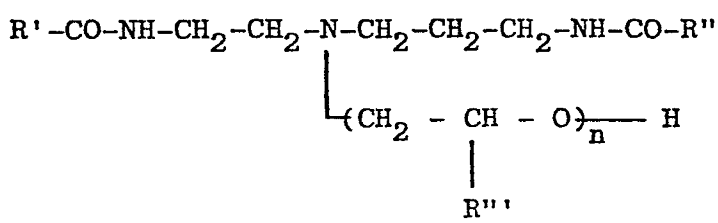

where R', R'', R''' and n have the meanings given above. This is then quaternised with an alkylating agent YX to give a quaternary ammonium compound with the following structure:-

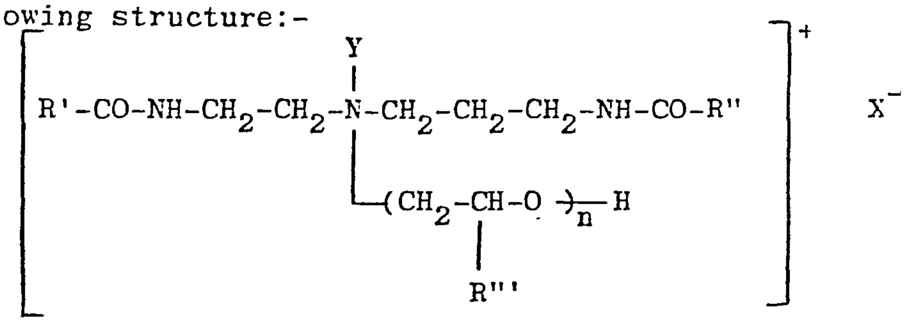

where Y and X have the meanings given above.

The following Examples illustrate the invention.

Example 1

600 grms of tallow fatty acid and 36 grms of oleic acid were charged into a glass reactor fitted with an azeotropic distillation facility. 137 grms of amino propyl ethylene diamine were added to the above followed by 100 grms of toluene. The whole was reacted at a temperature of between 70°C and 150°C during which water of reaction was removed by distillation. Reaction was continued until the residual acid value was less than 5.0 . At this stage, vacuum was applied and the temperature was raised to effect cyclisation to imidazoline and to remove toluene. Conversion to imidazoline was followed by wet analytical or infra-red techniques. The cyclisation was stopped when the imidazoline content reached a minimum of 90%. The product was discharged and allowed to cool.

275 grms of the above product was charged into a clean dry flask followed by 109.5 grms of iso-propanol and 1.5 grms of the chelating agent Nervanid FF. (Nervanid FF is sodium N-dihydroxy ethyl glycinate). This mixture was stirred for 15 minutes. 53.4 grms of dimethyl sulphate were then added over a period of 2 hours holding

the temperature between 40°C and 70°C . 6.0 grms of hydrogen peroxide were then added and the whole stirred. The pH of the product was then adjusted to between 5 and 7 by addition of dimethyl sulphate.

Properties of the Product

| | |
|---|---|
| Solids content : | 75% |
| pH: | 5 - 7 |
| Set Point : | 7°C |
| Colour (Gardner): | 6 |

Example 2

544 grms of tallow fatty acid were charged into a glass reactor fitted with an azeotropic distillation facility. 117 grms of amino propyl ethylene diamine were added to the above followed by 100 grms of toluene. The whole was reacted at a temperature of between 70°C and 130°C during which time water of reaction was removed by distillation. Reaction was continued until the residual acid value was less than 5.0. At this stage, the azeotropic distillation was stopped and normal distillation was commenced. Vacuum was applied and the toluene and residual water were removed keeping the temperature below 150°C. The product was cooled.

To 625 grms of the above product, 44 grms of ethylene oxide were added and the whole reacted at 30 - 40°C for one hour. 265 grms of iso-propanol were then added and the whole stirred for 15 minutes. 1.5 grms of Nervanid FF were then added followed by the addition of 126 grms of dimethyl sulphate over a period of 2 hours at a temperature of between 40°C and 70°C. 5.5 grms of hydrogen peroxide were then added and the pH of the product was adjusted to between 5.0 and 7.0 with dimethyl sulphate.

Properties of the product

| | |
|---|---|
| Solids content : | 75% |
| pH : | 5.0 - 7.0 |
| Set point: | 4°C |
| Colour (Gardner) : | 7 |

Example 3

600 grms of oleic acid were charged into a glass reactor fitted with an azeotropic distillation facility. 125.4 grms of amino propyl ethylene diamine were added to the above followed by 135 grms of toluene. The whole was reacted at a temperature of between 70°C and 150°C during which water of reaction was removed by distillation. Reaction was continued until the residual acid value was less than 5.0. At this stage, vacuum was applied and the temperature was raised to effect cyclisation to imidazoline and to remove toluene. Conversion to imidazoline was followed by wet analytical or infra-red techniques. The cyclisation was stopped when the imidazoline content reached a minimum of 90%. The product was discharged and allowed to cool.

500 grms of the above product was charged into a clean dry flask followed by 202 grms of iso-propanol and 2.8 grms of the chelating agent Nervanid FF. (Nervanid FF is sodium N-dihydroxy ethyl glycinate). This mixture was stirred for 15 minutes. 105.9 grms of dimethyl sulphate were then added over a period of 2 hours holding the temperature between 40°C and 70°C. 11.2 grms of hydrogen peroxide were then added and the whole stirred. The pH of the product was then adjusted to between 5 and 7 by addition of dimethyl sulphate.

Properties of the Product

Solids Content: 75%

pH: 5 - 7

Set Point: $< -5$°C

Colour (Gardner): 8.

The improved properties of the products of the present invention are illustrated in the following performance tests.

Softness Properties

A number of 100% cotton nappies of size 60 cm x 60 cm were washed to desize them. Four of the nappies were then rinsed with water containing 0.1% of one of the fabric softeners under test and dried in a tumble drier for a set time at a set temperature. The experiment was repeated for each of the other fabric softeners using a different set of four nappies each time. After drying, the nappies were folded and each set of four was assessed for 'handle' by a panel of five people. After the assessment, a strip of cotton (30 cm x 2.5 cm) was removed from two separate nappies from each set of four nappies. These strips were then used in the re-wetting tests to be described hereinafter. After the removal of these strips, the nappies were then rinsed with water and treated again with 0.1 % of the appropriate fabric softener and dried. Again strips (30 cm x 2.5 cm) were removed from the same nappies as the previous strips and tested for re-wetting properties. The nappies were tested in this way four times. In addition to the products of Examples 1, 2 and 3, three competitive fabric softeners were included in the test program along with an untreated set of nappies. The results of the assessment panel were statistically analysed and the products are listed in order of the "softening power" in Table 1 below :-

TABLE 1

| No. | Product | |
|---|---|---|
| 1 | Example 1 | 1-methyl-1-tallow amido propyl-2-tallow imidazolinium metho sulphate |
| 2 | Example 3 | 1-methyl-1-oleyl amido propyl-2-oleyl imidazolinium metho sulphate. |
| 3 | Example 2 | N-tallow amido ethyl N-tallow amido propyl N-ethoxylated methyl ammonium metho sulphate |
| 4 | Product B | 1-methyl-1-tallow amido ethyl-2-tallow imidazolinium metho sulphate (Varisoft 475) |
| 5 | Product C | 1-methyl-1-tallow-amido ethyl-2-tallow imidazolinium metho sulphate (Alkaquat T 75) |
| 6 | Product A | dimethyl di-H-tallow ammonium chloride (Arquad 2HT 75) |
| 7. | Untreated | |

Rewetting Properties

The rewetting properties were investigated using a standard "wicking" test carried out on the strips removed after each treatment. Each strip was dipped into water containing a dye and the height which the water attained was measured against time. Thetest was conducted at a temperature of 20°C. The results are shown in Tables 2, 3 and 4 below. In each case, the heights given are in millimetres and are the average figures obtained from two tests.

Table 2

Wicking test after 1st Treatment with fabric softener

| Time mins | Blank | Example 1 | Example 2 | Example 3 | Product A | Product B | Product C |
|---|---|---|---|---|---|---|---|
| 1 | 30 | 40 | 30 | 30 | 35 | 23 | 28 |
| 5 | 38 | 46 | 35 | 38 | 40 | 25 | 32 |
| 10 | 46 | 48 | 42 | 40 | 44 | 30 | 36 |
| 20 | 58 | 50 | 48 | 45 | 46 | 30 | 40 |
| 30 | 68 | 54 | 54 | 50 | 50 | 34 | 44 |
| 150 | 80 | 62 | 60 | 60 | 55 | 45 | 50 |

Table 3

Wicking test after 2nd Treatment with fabric softeners

| Time mins | Blank | Example 1 | Example 2 | Example 3 | Product A | Product B | Product C |
|---|---|---|---|---|---|---|---|
| 1 | 33 | 30 | 28 | 28 | 28 | 25 | 30 |
| 5 | 41 | 40 | 34 | 35 | 35 | 28 | 34 |
| 10 | 48 | 45 | 41 | 40 | 40 | 34 | 38 |
| 20 | 56 | 50 | 45 | 45 | 42 | 37 | 43 |
| 30 | 66 | 55 | 51 | 50 | 50 | 45 | 48 |
| 150 | 78 | 61 | 58 | 55 | 55 | 52 | 54 |

Table 4

Wicking Test after 4th Treatment with fabric softener

| Time mins | Blank | Example 1 | Example 2 | Example 3 | Product A | Product B | Product C |
|---|---|---|---|---|---|---|---|
| 1 | 32 | 41 | 31 | 30 | 30 | 25 | 32 |
| 5 | 37 | 46 | 34 | 35 | 35 | 31 | 35 |
| 10 | 45 | 49 | 43 | 40 | 41 | 37 | 38 |
| 20 | 56 | 52 | 48 | 48 | 47 | 44 | 44 |
| 30 | 66 | 58 | 55 | 55 | 53 | 49 | 48 |
| 150 | 76 | 66 | 61 | 60 | 59 | 54 | 54 |

Dispersibility Properties

4 grms of one of the fabric softeners were added to 100 grms of water. (Both the water and fabric softener had been pre-conditioned to 5°C). The mixture was slowly stirred and the time taken for, and ease of, dispersion were noted. Each fabric softener was tested in turn. The test was then repeated with the water and fabric softeners pre-conditioned to 20°C.

The results of the tests were as follows:-

| Product | 5°C | 20°C |
|---|---|---|
| Example 1 | very easily dispersed | very easily dispersed |
| Example 3 | very easily dispersed | very easily dispersed |
| Example 2 | easily dispersed | easily dispersed |
| *Product A | not dispersed | not dispersed |
| Product B | difficult to disperse | difficult to disperse |
| Product C | difficult to disperse | difficult to disperse |

* required warm water to disperse

Set Point Comparison

The set points of the products were as follows:

| Product | Set Point |
|---|---|
| Example 1 - | 7°C |
| Example 2 - | 4°C |
| Example 3 - | < -5°C |
| Product A - | 40°C |
| Product B - | 12°C |
| Product C - | 17°C |

Example 4

600 grms of oleic acid were charged into a glass reactor fitted with an azeotropic distillation facility. 125.4 grms of amino propyl ethylene diamine were added to the above followed by 135 grms of toluene. The whole was reacted at a temperature of between 70°C and 150°C during which water of reaction was removed by distillation. Reaction was continued until the residual acid value was less than 5.0. At this stage, vacuum was applied and the temperature was raised to effect cyclisation to imidazoline and to remove toluene. Conversion to imidazoline was followed by wet analytical or infra-red techniques. The cyclisation was stopped when the imidazoline content reached a minimum of 90%. The product was discharged and allowed to cool.

500 grms of the above product was charged into a clean dry flask followed by 2.8 grms of the chelating agent Nervanid FF. (Nervanid FF is sodium N-dihydroxy ethyl glycinate). This mixture was stirred for 15 minutes. 105.9 grms of dimethyl sulphate were then added over a period of 2 hours holding the temperature between 40°C and 70°C. 11.2 grms of hydrogen peroxide were then added and the whole stirred. The pH of the product was then adjusted to between 5 and 7 by addition of dimethyl sulphate.

The product had useful fabric softening properties.

Example 5

600 grms of palm oil and 36 grms of oleic

acid were charged into a glass reactor fitted with an azeotropic distillation facility. 137 grms of amino propyl ethylene diamine were added to the above followed by 100 grms of toluene. The whole was reacted at a temperature of between 70°C and 150°C. Reaction was continued until the residual acid value was less than 5.0. At this stage, vacuum was applied and the temperature was raised to effect cyclisation to imidazoline and to remove toluene. Conversion to imidazoline was followed by wet analytical or infra-red techniques. The cyclisation was stopped when the imidazoline content reached a minimum of 90%. The product was discharged and allowed to cool.

275 grms of the above product was charged into a clean dry flask followed by 109.5 grms of isopropanol and 1.5 grams of the chelating agent Nervanid FF. (Nervanid FF is sodium N-dihydroxy ethyl glycinate). This mixture was stirred for 15 minutes. 53.4 grms of dimethyl sulphate were then added over a period of 2 hours holding the temperature between 40°C and 70°C. 6.0 grms of hydrogen peroxide were then added and the whole stirred. The pH of the product was then adjusted to between 5 and 7 by addition of dimethyl sulphate.

The product had useful fabric softening properties.

Example 6

600 grms of tallow fatty acid and 36 grms of oleic acid were charged into a glass reactor fitted with an azeotropic distillation facility. 137 grms of amino propyl ethylene diamine were added to the above followed by 100 grms of toluene. The whole was reacted at a temperature of between 70°C and 150°C. Reaction was continued until the residual acid value was less than 5.0. At this stage, vacuum was applied

and the temperature was raised to effect cyclisation to imidazoline and to remove toluene. Conversion to imidazoline was followed by wet analytical or infra-red techniques. The cyclisation was stopped when the imidazoline content reached a minimum of 90%. The product was discharged and allowed to cool.

275 grms of the above product was charged into a clean dry flask followed by 109.5 grms of iso-propanol and 1.5 grams of the chelating agent Nervanid FF. (Nervanid FF is sodium N-dihydroxy ethyl glycinate). This mixture was stirred for 15 minutes. 53.4 grms of benzyl chloride were then added over a period of 2 hours holding the temperature between 40°C and 70°C. 6.0 grms of hydrogen peroxide were then added and the whole stirred. The pH of the product was then adjusted to between 5 and 7 by addition of benzyl chloride.

The product had useful fabric softening properties.

Example 7

544 grms of sunflower oil fatty acid were charged into a glass reactor fitted with an azeotropic distillation facility. 117 grms of amino propyl ethylene diamine were added to the above followed by 100 grms of toluene. The whole was reacted at a temperature of between 70°C and 130°C during which time water of reaction was removed by distillation. Reaction was continued until the residual acid value was less than 5.0. At this stage, the azeotropic distillation was stopped and normal distillation was commenced. Vacuum was applied and the toluene and residual water were removed keeping the temperature below 150°C. The product was cooled.

To 625 grms of the above product, 44 grams of ethylene oxide were added and the whole reacted at 30-40°C for one hour. 265 grams of isopropanol were then added and the whole stirred for 15 minutes.

1.5 grms of Nervanid FF were then added followed by the addition of 126 grms of dimethyl sulphate over a period of 2 hours at a temperature of between 40°C and 70°C. 5.5 grms of hydrogen peroxide were then added and the pH of the product was then adjusted to between 5.0 and 7.0 with dimethyl sulphate.

The product had useful fabric softening properties.

Example 8

544 grms of tallow fatty acid were charged into a glass reactor fitted with an azeotropic distillation facility. 117 grms of amino propyl ethylene diamine were added to the above followed by 100 grms of toluene. The whole was reacted at a temperature of between 70°C and 130°C during which time water of reaction was removed by distillation. Reaction was continued until the residual acid value was less than 5.0. At this stage, the azeotropic distillation was stopped and normal distillation was commenced. Vacuum was applied and the toluene and residual water were removed keeping the temperature below 150°C. The product was cooled.

To 625 grms of the above product, 44 grams of ethylene oxide were added and the whole reacted at 30-40°C for one hour. 265 grams of isopropanol were then added and the whole stirred for 15 minutes. 1.5 grms of Nervanid FF were then added followed by the addition of 126 grms of benzyl chloride over a period of 2 hours at a temperature of between 40°C and 70°C. 5.5 grms of hydrogen peroxide were then added and the pH of the product was then adjusted to between 5.0 and 7.0 with benzyl chloride.

The product had useful fabric softening properties.

Example 9

440 grms of coconut oil fatty acid were

charged into a glass reactor fitted with an azeotropic distillation facility. 125.4 grms of amino propyl ethylene diamine were added to the above followed by 135 grms of toluene. The whole was reacted at a temperature of between 70°C and 150°C during which water of reaction was removed by distillation. Reaction was continued until the residual acid value was less than 5.0. At this stage, vacuum was applied and the temperature was raised to effect cyclisation to imidazoline and to remove toluene. Conversion to imidazoline was followed by wet analytical or infra-red techniques. The cyclisation was stopped when the imidazoline content reached a minimum of 90%. The product was discharged and allowed to cool.

390 grms of the above product was charged into a clean dry flask followed by 202 grms of isopropanol and 2.8 grms of the chelating agent Nervanid FF. (Nervanid FF is sodium N-dihydroxy ethyl glycinate). This mixture was stirred for 15 minutes. 105.9 grms of dimethyl sulphate were then added over a period of 2 hours holding the temperature between 40°C and 70°C. 11.2 grms of hydrogen peroxide were then added and the whole stirred. The pH of the product was then adjusted to between 5 and 7 with dimethyl sulphate.

The product had useful fabric softening properties.

Example 10

620 grms of stearyl chloride were charged into a glass reactor fitted with an azeotropic distillation facility. 125.4 grms of amino propyl ethylene diamine were added to the above followed by 135 grms of toluene. The whole was reacted at a temperature of between 70°C and 150°C. Reaction was continued until the residual acid value was less than 5.0. At

this stage, vacuum was applied and the temperature was raised to effect cyclisation to imidazoline and to remove toluene. Conversion to imidazoline was followed by wet analytical or infra-red techniques. The cyclisation was stopped when the imidazoline content reached a minimum of 90%. The product was discharged and allowed to cool.

500 grms of the above product was charged into a clean dry flask followed by 202 grms of iso-propanol and 2.8 grms of the chelating agent Nervanid FF. (Nervanid FF is sodium N-dihydroxy ethyl glycinate). This mixture was stirred for 15 minutes. 105.9 grms of dimethyl sulphate were then added over a period of 2 hours holding the temperature between 40°C and 70°C. 11.2 grms of hydrogen peroxide were then added and the whole stirred. The pH of the product was then adjusted to between 5 and 7 with dimethyl sulphate.

The product had useful fabric softening properties.

In each of the foregoing examples, the processing was carried out in the presence of nitrogen as an inert gas to maintain the colour of the product.

CLAIMS:

1. A composition of matter which is the product obtained by reacting together aminopropyl ethylene diamine with a fatty acid or amide-forming derivative thereof to form a fatty diamide having a secondary amine group, converting the secondary amine group into a tertiary amine group by imidazoline formation or by alkoxylation, and quaternising the tertiary amine group.

2. A composition of matter having the general formula

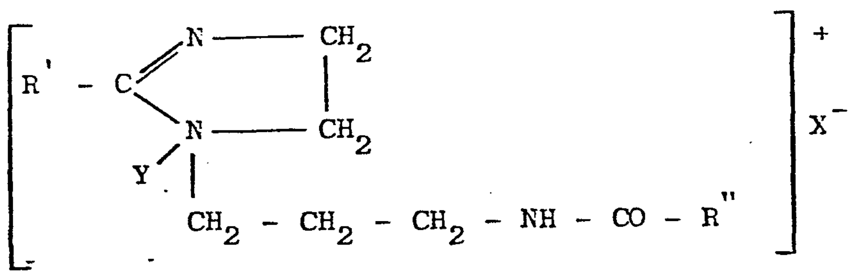

wherein R' and R" are fatty acid radicals having from 12 to 18 carbon atoms, Y is an alkyl or aryl radial, and $X^-$ is a halide or metho sulphate ion.

3. A composition of matter as claimed in claim 2 and having the formula

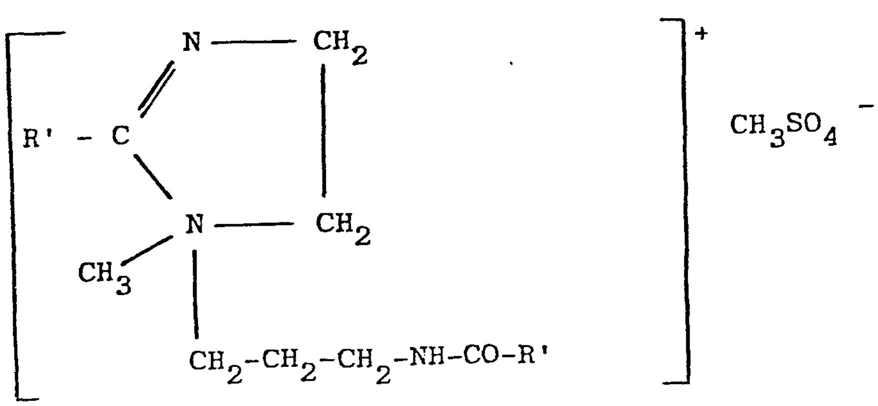

where R' is an oleic radical.

4. A composition of matter as claimed in claim 2 and having the formula

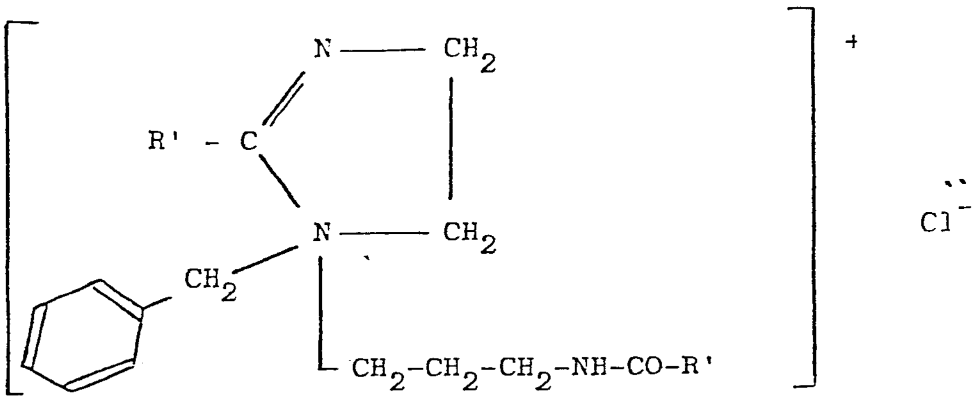

where R' is an oleic or stearyl radical.

5. A composition of matter having the general formula

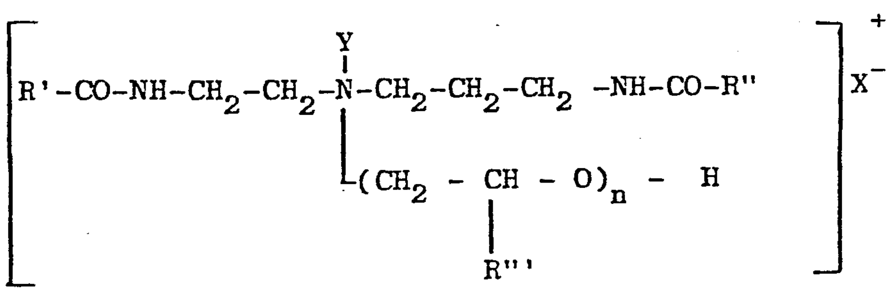

wherein R' and R" are fatty acid radicals having from 12 to 18 carbon atoms, Y is an alkyl or aryl radical, $X^-$ is a halide or metho sulphate ion, R"' is a hydrogen atom or a methyl group, and n is an integer of from 1 to 10.

6. A composition of matter as claimed in claim 5 and having the formula

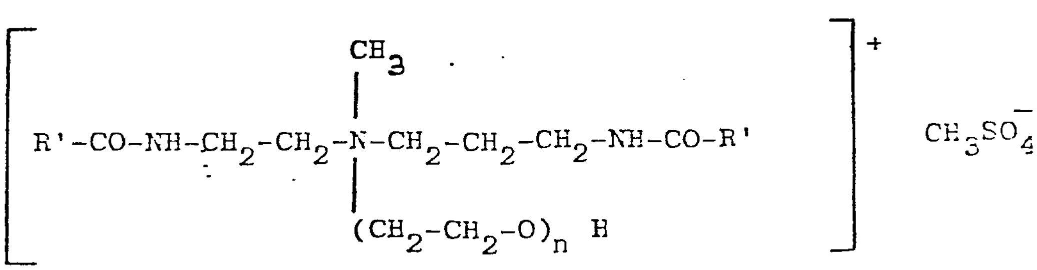

where R' is an oleic or stearyl radical.

7. A composition of matter as claimed in claim 5 and having the formula

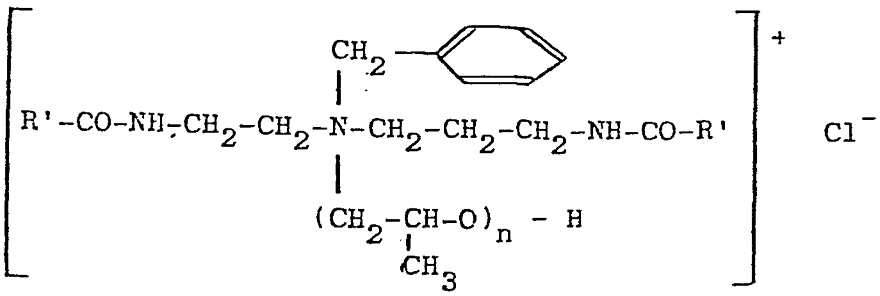

where R' is an oleic or stearyl radical

8. A process for producing a composition of matter by reacting together (i) a compound containing at least two primary amine groups and (ii) a fatty acid or amide-forming derivative thereof to form a fatty diamide having a secondary amine group, converting the secondary amine group into a tertiary amine group by imidazoline formation or by alkoxylation, and quaternising the tertiary amine group characterised in that said compound is amino propyl ethylene diamine.

9. A process in accordance with claim 8 wherein the fatty acid is oleic acid or stearic acid.

10. The use of a composition, as claimed in any one of claims 1 to 7, as a fabric softener.

0186967

European Patent Office

# EUROPEAN SEARCH REPORT

Application number

EP 85 30 8565

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE-A-2 641 286 (TEXACO)<br>--- | | C 07 D 233/16<br>C 07 C 103/44<br>D 06 M 13/46<br>C 11 D 1/62 |
| A | EP-A-0 023 334 (HOECHST)<br>--- | | |
| A | EP-A-0 023 333 (HOECHST)<br>--- | | |
| A | GB-A-1 395 663 (VAN DIJK)<br>--- | | |
| A | EP-A-0 082 456 (HOECHST)<br>--- | | |
| A | EP-A-0 082 457 (HOECHST)<br>--- | | TECHNICAL FIELDS SEARCHED (Int. Cl.4) |
| A | FR-A-2 033 133 (BASF)<br>----- | | C 07 D 233/00<br>C 07 C 103/00<br>D 06 M 13/00<br>C 11 D 1/00 |

The present search report has been drawn up for all claims

| Place of search | Date of completion of the search | Examiner |
|---|---|---|
| THE HAGUE | 18-03-1986 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document